# EUROPEAN PATENT SPECIFICATION

(11) **EP 3 383 510 B1**
(45) Date of publication and mention of the grant of the patent: **29.04.2020**
(21) Application number: 16805106.8
(22) Date of filing: 01.12.2016
(51) Int. Cl.: A61B 5/11, A63B 71/06, A63B 24/00, A61B 5/00, G04G 21/02, H04B 1/3827

(54) **ACTIVITY IDENTIFICATION AND TRACKING**
AKTIVITÄTSIDENTIFIZIERUNG UND -VERFOLGUNG
IDENTIFICATION ET SUIVI D'ACTIVITÉ

(30) Priority: 01.12.2015 US 201562261489 P
(43) Date of publication of application: 10.10.2018
(73) Proprietor: Koninklijke Philips N.V., 5656 AG Eindhoven (NL)
(72) Inventor: KRANS, Jan, Martijn, 5656 AE Eindhoven (NL); HÄRMÄ, Aki, Sakari, 5656 AE Eindhoven (NL); VAN DANTZIG, Saskia, 5656 AE Eindhoven (NL)
(74) Representative: Coops, Peter
(86) International application number: PCT/EP2016/079414
(87) International publication number: WO 2017/093391

(56) References cited:
- EP-A1- 1 897 598
- EP-A2- 2 687 114
- WO-A1-2015/027133
- WO-A1-2015/164944
- WO-A2-2012/061438
- US-A1- 2007 219 059
- US-B2- 8 821 350

## Description

### FIELD OF THE INVENTION

The present invention is generally related to activity tracking and, more particularly, is related to wearable devices that recognize and track activity.

### BACKGROUND OF THE INVENTION

A large variety of activity trackers, such as physical activity trackers, is being offered on the market. Such activity trackers may be worn as a bracelet or wristband, and include one or more sensors ranging from a single accelerometer to additional sensors such as height and/or heart rate sensors. For instance, these activity trackers may provide information relevant to fitness or health, such as steps taken during a day or distance covered. For sports activities, U.S. Patent No. 8,353,791 describes a system containing a data logger worn by a player and sensors fitted to the players footwear, glove or a bat, stick, club, or racquet to register a kick or ball strike. Additional sensors on the data logger, such as gyro and accelerometer sensors, can be used to provide diagnostic information about a golf swing for later analysis or entertainment. For instance, the gyroscope can tell how fast the club was swung during a stroke and its appropriateness for the shot.

In WO2015027133A1 it is disclosed that athletic activity may be tracked and monitored while providing encouragement and maintaining an individual's interest in continuing to perform athletic activity. For example, energy expenditure values and energy expenditure intensity values may be calculated based on the duration and type of activity performed by an individual. These values and other movement data may be displayed on an interface in a manner to motivate the individual and maintain the individual's interest. Other individuals (e.g.,friends) may also be displayed on an interface through which a user's progress is tracked. This may allow the user to also view the other individuals' progress toward completing an activity goal and/or challenge.

### SUMMARY OF THE INVENTION

The present invention relates to a wearable device as claimed in claim 1; a method of operating the wearable device as claimed in claim 14; and a computer program product as claimed in claim 15. One object of the present invention is that a wearable device can automatically identify a type of activity.

Another object of the present invention is that a wearable device can measure one or more physiological and/or behavioral parameters based on the identified type of activity.

An embodiment of the present invention provides a wearable device that comprises a wireless receiver circuit for wirelessly receiving a local signal emanating outside of the device, the signal comprising information indicative of a type of activity selectable from among a plurality of types of activities, a plurality of sensors for sensing one or more physiological and behavioral parameters, and a processing circuit for automatically determining the type of activity based on the information in the received signal and for receiving data corresponding to the one or more physiological and behavioral parameters from one or more of the plurality of sensors based on the determination.

In an embodiment, the determined type of activity is related to a person wearing the wearable device, and the one or more physiological and/or behavioral parameters are associated with the person's body function and biomechanics (e.g., movement of all or a part of the person's body, such as a person's kicking motion or swing). Further, the one or more physiological and behavioral parameters may be used to derive additional parameters. The identification of the type of activity enables a more accurate interpretation of the person's activity and well-being. In contrast, there is a limited ability for today's activity trackers to distinguish between rudimentary types of activity. That is, walking may be distinguished from running or biking or manual input may be used after the activity during synch-up to a web-server or application for purposes of responding to predetermined questions regarding the type of activity a person is engaged in.

In an embodiment, the wireless receiver circuit for wirelessly receiving the signal coded with identifier information according to radio frequency identification technology, near field communication technology, or Bluetooth technology. The decoding of the signal enables the identification of a type of activity without requiring human intervention.

In an embodiment, the processing circuit for executing one of a plurality of selectable algorithms stored in the memory based on determining the type of activity. The fine tuning of algorithms specific to the identified type of activity enables a more accurate determination of information or parameters that are based, directly or indirectly (e.g., derived), on the data corresponding to the physiological and/or behavioral parameters, such as energy expenditure computations.

In an embodiment, further comprising an analog-to-digital converter, the processing circuit further for causing the analog-to-digital converter to operate at a higher sampling rate for data acquisition based on determining the type of activity. The ability to switch to a higher sampling rate improves the accuracy of data acquisition, and enables the wearable device to operate at lower power when an activity is not detected so as to reduce power consumption and/or the size of the power source.

In an embodiment, the processing circuit further for causing the provision of feedback responsive to determining the type of activity. Such feedback provides the person with confidence that the wearable device is operating with fidelity.

In general, certain embodiments of a wearable device are disclosed that automate the detection and determination of a type of activity, and both measure physiological and/or behavioral parameters and compute information corresponding to measured physiological and/or behavioral parameters based on the determined type of activity.

These and other aspects of the invention will be apparent from and elucidated with reference to the embodiment(s) described hereinafter.

### BRIEF DESCRIPTION OF THE DRAWINGS

Many aspects of the invention can be better understood with reference to the following drawings, which are diagrammatic. The components in the drawings are not necessarily to scale, emphasis instead being placed upon clearly illustrating the principles of the present invention. Moreover, in the drawings, like reference numerals designate corresponding parts throughout the several views.
FIG. 1 is a schematic diagram that illustrates an example system in which a wearable device is used in accordance with an embodiment of the invention.
FIG. 2 is a block diagram that illustrates circuitry for an example wearable device in accordance with an embodiment of the invention.
FIGS. 3A-3C are schematic diagrams that illustrate several example uses for a wearable device in accordance with an embodiment of the invention.
FIG. 4 is a flow diagram that illustrates a method for operating a wearable device in accordance with an embodiment of the invention.

### DETAILED DESCRIPTION OF EMBODIMENTS

Disclosed herein are certain embodiments of a wearable device and corresponding method that enables automatic and reliable detection and identification of a specific type of sports activity, exercise (also referred to as fitness) activity, recreational activity (e.g., darts, billiards, etc.), household activity (e.g., painting, cleaning, etc.), social activity, and/or even sedentary activity (e.g., sleep), among other types of activities. The automating of the detection and identification process removes or mitigates the burden on a user to enter such information manually, and also increases the activity-discernment capability over fitness or activity trackers today that measure physiological parameters, such as heart rate, respiration, etc. Processing circuitry in an embodiment of a wearable device is also operatively enhanced to improve physiological and/or behavioral measurements compared to those existing in the market today through real-time use of more activity-specific algorithms and/or sensors and more robust processing.

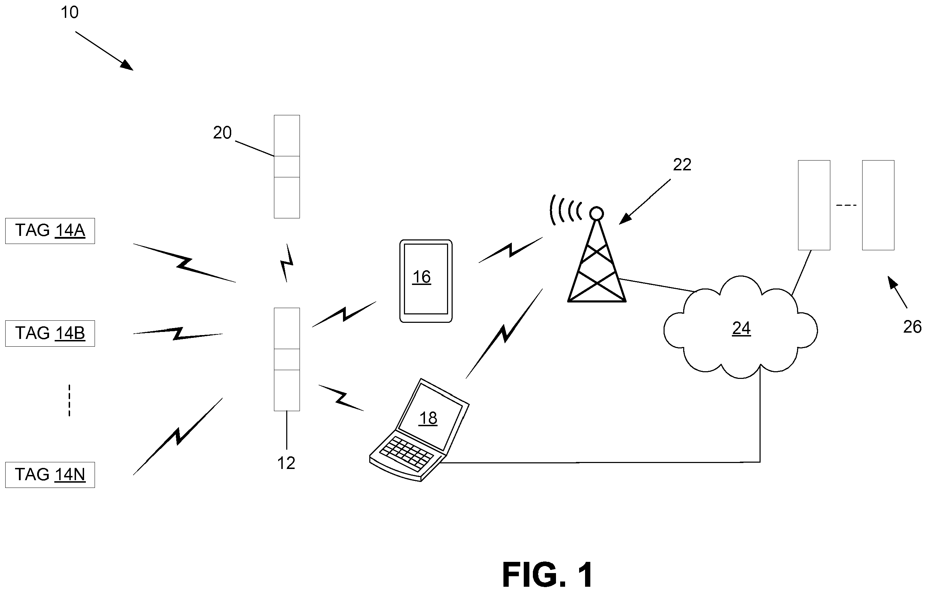

Attention is directed to FIG. 1, which illustrates an example system 10 in which an embodiment of a wearable device 12 may be implemented. It should be appreciated by one having ordinary skill in the art in the context of the present disclosure that the system 10 is one example among many, and that some embodiments of wearable devices 12 may be used in systems with fewer, greater, and/or different components that those depicted in FIG. 1. The system 10 comprises a plurality of devices that enable communication of information throughout one or more networks. The depicted system 10 comprises one or more tags 14 (e.g., 14A-14N) that each comprises a unique tag identifier and activity/apparatus identifier, and which are in wireless communication with the wearable device 12. The wearable device 12 is further communicatively coupled to one or more electronic devices associated with the system 10, including a phone 16, a computer 18, and/or another wearable device 20. It should be appreciated that although each electronic device is listed in the singular, some implementations may utilize different quantities for each of the electronic devices 16-20. The system 10 further comprises a cellular network 22 that enables the communication of information between the phone 16 and the computer 18 (e.g., equipped with a cellular modem card) and another network, such as a wide area network 24. The computer 18 may also be coupled via a wired connection, such as coaxial cable, twisted pair, and/or copper wiring, to the wide area network 24. The system 10 further comprises one or more computing devices, such as one or more server devices 26 that may host web services and/or data storage for the rest of the system 10.

The tags 14 may be configured as one or any combination of radio frequency identification (RFID) tags/transponders, Bluetooth transceivers, and/or near field communication (NFC) transceivers/transponders. For instance, when one or more of the tags 14 are embodied as RFID tags, the RFID tag may comprise a semiconductor (e.g., silicon) integrated circuit or microchip and one or more antennas that enable the RFID tag 14 to receive and respond to radio frequency (RF) signals transmitted by the wearable device 12. The RFID tag 14 may be of an active type (e.g., on-board or re-usable power source) or passive type (e.g., energized by radio energy provided in the signal transmitted by the wearable device 12). The RFID tag 14 further comprises memory, such as non-volatile memory, which is used to store information embodied as an identifier of the tag 14 and of an identifier of an apparatus to which the RFID tag 14 is attached. In some embodiments, the identifier may be of the type of activity (e.g., through logical association with the apparatus) for which the apparatus is normally associated. For instance, for active-type tags 14, RFID reader functionality in the wearable device 12 may generate a modulated RF signal that is communicated to the RFID tag 14. The RFID tag 14 provides a modulated RF response signal having information coded in the signal, the information corresponding to the tag identifier and the identifier of the apparatus to which the RFID tag 14 is attached and which is stored in memory of the RFID tag 14. For passive-type tags, RFID reader functionality in the wearable device 12 provides an unmodulated, continuous wave signal to activate and power the RFID tag 14, wherein in one embodiment, a back-scattered signal from the RFID tag 14 is coded with the stored identifiers. The RFID reader functionality in the wearable device 12 demodulates and decodes the information (e.g., identifiers) to automatically determine the type of apparatus (and/or the type of activity associated with the apparatus).

For tags 14 embodied as NFC tags, communication is generally achieved within 10 centimeters (cm) between the reader and tag 14 and operation is in an unregulated (at least in some jurisdictions) RF band of 13.56 MHz. Like the RFID tags 14, the NFC tag 14 may operate in active (providing an RF field) or passive (e.g., energized by the wearable device 12) modes. It is noted that NFC is compatible with some passive-RFID infrastructures. The NFC tag 14 comprises an antenna and memory (e.g., non-volatile memory), the latter which stores information in the form of a tag identifier and identifier of the attached apparatus and/or associated activity of the attached apparatus. Communication between the tag 14 and the wearable device 12 is in the form of a modulated signal with information coded (e.g., modified Miller coding or Manchester coding) according to the stored identifiers, with active mode involving each of the wearable device 12 and tag 14 alternating their respective RF fields and in passive mode (where the tag 14 is alternatively referred to as a transponder) the field generated by the wearable device 12 modulated by the identifiers stored in the NFC tag 14. Further, in passive mode, the NFC tag 14 uses the RF field provided by the wearable device 12 to energize the tag 14.

Tags 14 embodied as Bluetooth transceivers utilize UHF radio waves (e.g., 2.4 - 2.485 MHz) and frequency hopping spread spectrum (e.g., dividing transmitted data into packets and transmitting each packet among seventy-nine (79) different Bluetooth channels). The tag 14 and wearable device 12 operate in a master-slave configuration, with in one embodiment, the wearable device 12 serving as the master and the tag 14 as the slave, though not limited to this configuration. The tag(s) 14 and the wearable device 12 share a common master clock embedded, in one embodiment, in the wearable device 12.

The phone 16 may be embodied as a smartphone, mobile phone, cellular phone, laptop, personal digital assistant, tablet, pager, among other handheld computing/communication devices with telephony functionality. For the sake of example, assume the phone 16 is embodied as a smartphone. The smartphone 16 comprises at least two different processors, including a baseband processor and an application processor. The baseband processor comprises a dedicated processor for deploying functionality associated with a protocol stack, such as a GSM (Global System for Mobile communications) protocol stack. The application processor comprises a multi-core processor for providing a user interface and running applications. The baseband processor and application processor have respective associated memory (e.g., random access memory (RAM), Flash memory, etc.), peripherals, and a running clock.

More particularly, the baseband processor deploys functionality of a GSM protocol stack to enable the smartphone 16 to access one or a plurality of wireless network technologies, including WCDMA (Wideband Code Division Multiple Access), CDMA (Code division Multiple Access), EDGE (Enhanced Data Rates for GSM Evolution), GPRS (General Packet Radio Service), Zigbee (e.g., based on IEEE 802.15.4), Bluetooth, Wi-Fi (Wireless Fidelity, such as based on IEEE 802.11), and/or LTE (Long Term Evolution), among variations thereof and/or other telecommunication protocols, standards, and/or specifications. The baseband processor manages radio communications and control functions, including signal modulation, radio frequency shifting, and encoding. The baseband processor may comprise a GSM modem having one or more antennas, a radio (e.g., RF front end), and analog and digital baseband circuitry. The RF front end comprises a transceiver and a power amplifier to enable the receiving and transmitting of signals of a plurality of different frequencies, enabling access to the cellular network 22. The analog baseband is coupled to the radio and provides an interface between the analog and digital domains of the GSM modem. The analog baseband comprises circuitry including an analog-to-digital converter (ADC) and digital-to-analog converter (DAC), as well as control and power management/distribution components and an audio codec to process analog and/or digital signals received from the smartphone user interface (e.g., microphone, earpiece, ring tone, vibrator circuits, etc.). The ADC digitizes any analog signals for processing by the digital baseband processor. The digital baseband processor deploys the functionality of one or more levels of the GSM protocol stack (e.g., Layer 1, Layer 2, etc.), and comprises a microcontroller (e.g., microcontroller unit or MCU) and a digital signal processor (DSP) that communicate over a shared memory interface (the memory comprising data and control information and parameters that instruct the actions to be taken on the data processed by the application processor). The MCU may be embodied as a RISC (reduced instruction set computer) machine that runs a real-time operating system (RTIOS), with cores having a plurality of peripherals (e.g., circuitry packaged as integrated circuits) such as RTC (real-time clock), SPI (serial peripheral interface), I2C (inter-integrated circuit), UARTs (Universal Asynchronous Receiver/Transmitter), devices based on IrDA (Infrared Data Association), SD/MMC (Secure Digital/Multimedia Cards) card controller, keypad scan controller, and USB devices, GPRS crypto module, TDMA (Time Division Multiple Access), smart card reader interface (e.g., for the one or more SIM (Subscriber Identity Module) cards), timers, and among others. For receive-side functionality, the MCU instructs the DSP to receive, for instance, in-phase/quadrature (I/Q) samples from the analog baseband and perform detection, demodulation, and decoding with reporting back to the MCU. For transmit-side functionality, the MCU presents transmittable data and auxiliary information to the DSP, which encodes the data and provides to the analog baseband (e.g., converted to analog signals by the DAC). The application processor may be embodied as a System on a Chip (SOC), and supports a plurality of multimedia related features including web browsing, email, multimedia entertainment, games, etc. The application processor includes an operating system that enables the implementation of a plurality of user applications. For instance, the application processor may deploy one or more application program interfaces (APIs) that enable access to a cloud computing framework or other networks to provide remote data access/storage/processing, and through cooperation with an embedded operating system, access to calendars, location services, reminders, etc. For instance, cloud computing may be used for storage of user data for enabling remote coaching, remote medical analysis, etc. The application processor generally comprises a processor core (Advanced RISC Machine or ARM), multimedia modules (for decoding/encoding pictures, video, and/or audio), a graphics processing unit (GPU), wireless interfaces, and device interfaces. The wireless interfaces may include a Bluetooth or Zigbee module(s) that enables wireless communication with the wearable devices 12, 20 or other local devices, a Wi-Fi module for interfacing with a local 802.11 network, and a GSM module for access to the cellular network 22 and access to the wide area network 24. The device interfaces coupled to the application processor may include a respective interface for such devices as a screen display (e.g., LCD or Liquid Crystal Display), keypad, USB (Universal Serial Bus), SD/MMC card, camera, GPRS, Wi-Fi, GPS, and/or FM radios, memory, among other devices. It should be appreciated by one having ordinary skill in the art, in the context of the present disclosure, that variations to the above may be deployed in some embodiments to achieve similar functionality.

The computer 18 may be embodied as a laptop, personal computer, workstation, tablet, among other computing devices with communication capability. The computer 18 may be in wireless or wired (e.g., temporarily, such as via USB connection, or persistently, such as an Internet connection or local area network connection) communication with other devices. The computer 18 may include similar hardware and software/firmware to that described above for the phone 16 to enable access to wireless and/or cellular networks (e.g., through communication cards comprising radio and/or cellular modem functionality) and/or other devices (e.g., Bluetooth transceivers, NFC transceivers, etc.), such as wireless or (temporary) wired connection to the wearable devices 12, 20. In some implementations, the computer 18 may be coupled to the Internet through the plain old telephone service (POTS), using technologies such as digital subscriber line (DSL), asymmetric DSL (ADSL), and/or according to broadband technology that uses a coaxial, twisted pair, and/or fiber optic medium. Discussion of such communication functionality is omitted here for brevity. Generally, in terms of hardware architecture, the computer 18 includes a processor, memory, and one or more input and/or output (I/O) devices (or peripherals) that are communicatively coupled via a local interface. The local interface can be, for example but not limited to, one or more buses or other wired or wireless connections. The local interface may have additional elements, which are omitted for brevity, such as controllers, buffers (caches), drivers, repeaters, and receivers, to enable communications. Further, the local interface may include address, control, and/or data connections to enable appropriate communications among the aforementioned components.

The processor is a hardware device for executing software, particularly that stored in memory. The processor can be any custom made or commercially available processor, a central processing unit (CPU), an auxiliary processor among several processors associated with the computer 18, a semiconductor based microprocessor (in the form of a microchip or chip set), a macroprocessor, or generally any device for executing software instructions.

The memory can include any one or combination of volatile memory elements (*e.g.*, random access memory (RAM, such as DRAM, SRAM, SDRAM, etc.) and nonvolatile memory elements (e.g., ROM, hard drive, tape, CDROM, etc.). Moreover, the memory may incorporate electronic, magnetic, optical, semi-conductive, and/or other types of storage media. Note that the memory can have a distributed architecture, where various components are situated remote from one another, but can be accessed by the processor.

The software in memory may include one or more separate programs, each of which comprises an ordered listing of executable instructions for implementing logical functions. The software in the memory includes application software and a suitable operating system (O/S). The operating system may be embodied as a Windows operating system available from Microsoft Corporation, a Macintosh operating system available from Apple Computer, a UNIX operating system, among others. The operating system essentially controls the execution of other computer programs, and provides scheduling, input-output control, file and data management, memory management, and communication control and related services.

The I/O devices may include input devices, for example but not limited to, a keyboard, mouse, scanner, microphone, etc. Furthermore, the I/O devices may also include output devices, for example but not limited to, a printer, display, etc. Finally, the I/O devices may further include devices that communicate both inputs and outputs, for instance but not limited to, a modulator/demodulator (modem, such as to access another device, system, or network), a radio frequency (RF) or Bluetooth transceiver, a telephonic interface, a bridge, a router, etc. as indicated previously.

If the computer is a PC, workstation, or the like, the software in the memory may further include a basic input output system (BIOS). The BIOS is a set of essential software routines that initialize and test hardware at startup, start the O/S, and support the transfer of data among the hardware devices. The BIOS is stored in ROM so that the BIOS can be executed when the computer 18 is activated.

When the computer 18 is in operation, the processor is configured to execute software stored within the memory, to communicate data to and from the memory, and to generally control operations of the computer 18 pursuant to the software.

Software can be stored on any non-transitory computer readable medium for use by or in connection with any computer related system or method. In the context of this document, a computer readable medium comprises an electronic, magnetic, optical, electromagnetic, infrared, or semiconductor system, apparatus, device or means that can contain or store a computer program for use by or in connection with a computer related system or method. The software can be embodied in any non-transitory computer-readable medium for use by or in connection with an instruction execution system, apparatus, or device, such as a computer-based system, processor-containing system, or other system that can fetch the instructions from the instruction execution system, apparatus, or device and execute the instructions.

The cellular network 22 may include the necessary infrastructure to enable cellular communications by the phone 16 and optionally the computer 18. There are a number of different digital cellular technologies suitable for use in the cellular network 22, including: GSM, GPRS, CDMAOne, CDMA2000, Evolution-Data Optimized (EV-DO), EDGE, Universal Mobile Telecommunications System (UMTS), Digital Enhanced Cordless Telecommunications (DECT), Digital AMPS (IS-136/TDMA), and Integrated Digital Enhanced Network (iDEN), among others.

The wide area network 24 may comprise one or a plurality of networks that in whole or in part comprise the Internet. Access to the Internet by the phone 16 and/or computer 18 may be enabled through one or more server devices 26 and/or networks, include PSTN (public switched telephone networks), POTS, Integrated Services digital Network (ISDN), Ethernet, Fiber, DSL/ADSL, among others.

In one example operation involving the wearable device 12, and assuming passive RFID tags 14, the wearable device 12 receives wireless signals (e.g., backscattered signals) from one or more of the passive RFID tags 14. For instance, the wearable device 12 may poll for tags 14 periodically or based on detected movements of a subject upon which the wearable device 12. The wireless signals comprise coded information such as a tag identifier and an identifier of an apparatus to which the RFID tag(s) is attached. In one embodiment, the wearable device 12 associates the identifier of the apparatus with a specific activity associated with a subject and commences heightened processing (e.g., increased sampling rate) as well as selection of a subset of available sensors for measuring physiological and/or behavioral parameters. In some embodiments, the wearable device 12 further selects an algorithm that is tailored for use in measuring physiological or behavioral parameters from the subset of sensors of the subject associated with the specific, detected activity. In some embodiments, the wearable device 12 provides feedback of the heightened processing (e.g., via a tactile alert, audible sound, tone, etc.). The wearable device 12 communicates with the phone 16 (or computer 18 in some embodiments), such as according to Bluetooth communication technology. The phone 16 may provide feedback to the subject (e.g., in the form of visual display or aural or tactile feedback) about the measured physiological and/or behavioral parameters (e.g., summary) or information related to the physiological and/or behavioral parameters, or through execution of an application and API, communicate with one or more server devices 26 coupled to the wide area network 24 (e.g., arranged in a cloud computing arrangement), enabling the data corresponding to the measured physiological or behavioral parameters to be submitted for entry in a data structure for further processing and/or synthesis with other data. For instance, the submitted data may be used for inclusion into a chart or graphic or for evaluation by a remote coach that offers consultation to a person associated with the detected activity. Note that in some embodiments, the wearable device 12 may wirelessly communicate with the phone 16 for access to a list of activity/apparatus identifiers in lieu of, or in addition to, local storage of identifiers.

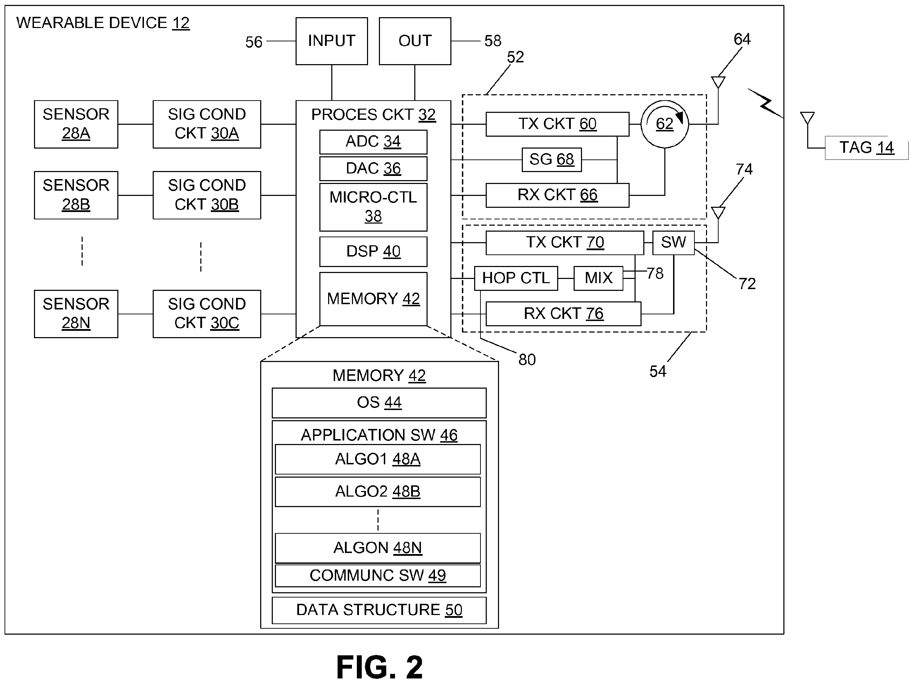

Having generally described an example system 10 in which an embodiment of a wearable device 12 may be implemented, as well as an example operation involving the wearable device 12, attention is directed to FIG. 2. FIG. 2 illustrates an embodiment of the example wearable device 12, and in particular, underlying circuitry and software (e.g., architecture) of the wearable device 12. It should be appreciated by one having ordinary skill in the art in the context of the present disclosure that the architecture of the wearable device 12 depicted in FIG. 2 is but one example, and that in some embodiments, additional, fewer, and/or different components may be used to achieve similar and/or additional functionality. In one embodiment, the wearable device 12 comprises a plurality of sensors 28 (e.g., 28A, 28B,...28N), signal conditioning circuits 30 (e.g., 30A, 30B,...30N) coupled respectively to the sensors 28, and a processing circuit 32 that receives the conditioned signals from the signal conditioning circuits 30. In one embodiment, the processing circuit 32 comprises an analog-to-digital converter (ADC) 34, a digital-to-analog converter (DAC) 36, a microcontroller 38 (e.g., MCU), a digital signal processor (DSP) 40, and memory 42. In some embodiments, the processing circuit 32 may comprise fewer or additional components than those depicted in FIG. 2. For instance, in one embodiment, the processing circuit 32 may consist of the microcontroller 38. The memory 42 comprises an operating system (OS) 44 and application software 46. The application software 46 comprises a plurality of algorithms 48 (e.g., application modules of executable code) to process the signals (and associated data) measured by the sensors 28, including a first algorithm (algo1) 48A, a second algorithm (algo2) 48B, up to an Nth algorithm (algoN) 48N. In one embodiment, the different algorithms 48 are tasked depending on the detected activity and the desired physiological and/or behavioral parameters for the detected activity, with the different algorithms using a different subset of sensors 28. The application software 46 also comprises communications software 49, such as that used to enable the wearable device 12 to operate according to one or more of a plurality of different communication technologies (e.g., RFID, NFC, Bluetooth, Wi-Fi, Zigbee, etc.). In some embodiments, the communications software 49 may be in separate or other memory. The memory 42 further comprises one or more data structures, such as data structure 50. In one embodiment, the processing circuit 32 is coupled to an identifier receiving circuit 52 and an external communications circuit 54. The identifier receiving circuit 52 serves to receive wireless signals from one or more tags 14 and forward information (e.g., a tag identifier and apparatus identifier) coded in the signal to the processing circuit 32 to enable the processing circuit 32 to identify the tag 14 and an apparatus to which the tag(s) 14 are attached (or identify an associated activity in which the subject using the apparatus is engaged). The identifier receiving circuit 52 is depicted as an RFID reader circuit, but in some embodiments, may be configured as any one or a combination of an NFC circuit (which can read some passive RFID tags 14) or a Bluetooth circuit. In some embodiments, the identifier receiving circuit 52 may include a combination of two or more of the RFID reader circuit, NFC circuit, and the Bluetooth circuit. The external communications circuit 54 serves to enable wireless communications between the wearable device 12 and other electronic devices, such as the phone 16, the laptop 18, and/or the wearable device 20 (FIG. 1). The external communications circuit 54 is depicted as a Bluetooth circuit, though not limited to this transceiver configuration. For instance, in some embodiments, the external communications circuit 54 may be embodied as any one or a combination of an NFC circuit, Wi-Fi circuit, transceiver circuitry based on Zigbee, among others such as optical or ultrasonic based technologies. In some embodiments, functionality of the identifier receiving circuit 52 and the external communications circuit 54 may be implemented using a single transceiver circuit (e.g., using a Bluetooth transceiver to receive coded information from the tags 14 and to receive from, and send information to, other electronic devices). Description of the components of the identifier receiving circuit 52 and the external communications circuit 54 is described further below. The processing circuit 32 is further coupled to input/output (I/O) devices or peripherals, such as an input interface 56 and output interface 58. Note that in some embodiments, functionality for one or more of the aforementioned circuits and/or software may be combined into fewer components/modules, or in some embodiments, further distributed among additional components/modules. For instance, the processing circuit 32 may be packaged as an integrated circuit that includes the microcontroller 38, the DSP 40, and memory 42, whereas the ADC 34 and DAC 36 may be packaged as a separate integrated circuit coupled to the processing circuit 32. In some embodiments, one or more of the functionality for the above-listed components may be combined, such as functionality of the DSP 40 performed by the microcontroller 38.

Referring first to the physiological and behavioral sensing functionality managed and coordinated by the processing circuit 32, the sensors 28 are selected to perform detection and measurement of a plurality of physiological and behavioral parameters, including heart rate, heart rate variability, heart rate recovery, blood flow rate, activity level, muscle activity (e.g., movement of limbs, repetitive movement, core movement, body orientation/position, power, speed, acceleration, etc.), muscle tension, blood volume, blood pressure, blood oxygen saturation, respiratory rate, perspiration, skin temperature, and body composition. The sensors 28 may be embodied as inertial sensors (e.g., gyroscopes, single or multi-axis accelerometers, such as those using piezoelectric, piezoresistive or capacitive technology in a microelectromechanical system (MEMS) infrastructure), flex and/or force sensors (e.g., using variable resistance), electromyographic sensors, electrocardiographic sensors (e.g., EKG, ECG) magnetic sensors, photoplethysmographic (PPG) sensors, bio-impedance sensors, and/or infrared proximity sensors, acoustic/ultrasonic/audio sensors, strain gauge, galvanic skin sensors/sweat, pH sensors, temperature sensors, pressure sensors, and photocells. In some embodiments, other types of sensors 28 may be used to facilitate health and/or fitness related computations, including a global navigation satellite systems (GNSS) sensor (e.g., global positioning system (GPS) sensor) to facilitate determinations of distance, speed, acceleration, location, altitude, etc., barometric pressure, humidity, outdoor temperature, etc. In some embodiments, GNSS functionality may be achieved via the external communications circuit 54.Note that in some embodiments, functionality for measuring physiological parameters may be combined using fewer sensors 28 than those listed above. Certain embodiments may use a subset of sensors 28 of the available sensors (e.g., fewer than all sensors) depending on the determined activity, and in some embodiments, the refresh rate, accuracy, or other sensor parameters may be altered depending on the detected activity.

The signal conditioning circuits 30 include amplifiers and filters, among other signal conditioning components, to condition the sensed signals including data corresponding to the sensed physiological parameters before further processing is implemented at the processing circuit 32. Though depicted in FIG. 2 as respectively associated with each sensor 28, in some embodiments, fewer signal conditioning circuits 30 may be used (e.g., shared for more than one sensor 28). In some embodiments, the signal conditioning circuits 30 (or functionality thereof) may be incorporated elsewhere, such as in the circuitry of the respective sensors 28 or in the processing circuit 32 (or in components residing therein). Further, although described above as involving unidirectional signal flow (e.g., from the sensor 28 to the signal conditioning circuit 30), in some embodiments, signal flow may be bi-directional. For instance, in the case of optical measurements, the microcontroller 38 may cause an optical signal to be emitted from a light source (e.g., light emitting diode(s) or LED(s)) in or coupled to the circuitry of the sensor 28, with the sensor 28 (e.g., photocell) receiving the reflected/refracted signals.

The identifier receiving circuit 52 is managed and controlled by the processing circuit 32. In the depicted embodiment, the identifier receiving circuit 52 is embodied as an RFID reader (or interrogator) circuit for reading a passive RFID tag 14. In one embodiment, the identifier receiving circuit 52 comprises a transmitter circuit 60, a directional coupler 62, an antenna 64, a receiver circuit 66, and a signal generator circuit 68. The transmitter circuit 60 and the receiver circuit 66 comprise components suitable for providing respective transmission and reception of an RF signal, including a modulator/demodulator, filters, and amplifiers. In some embodiments, one or more of the preceding functionality may be performed by the DSP 40 or microcontroller 38. The directional coupler 62 directs energy to and from the antenna 64. The signal generator circuit 68 may be embodied as an oscillating circuit and/or frequency synthesizer, as controlled by the processing circuit 32. Control for the identifier receiving circuit 52 may be implemented by the microcontroller 38, the DSP 40, or a combination of both. In some embodiments, a separate reader controller may be implemented that is under the supervision of the microcontroller 38.

The passive RFID tag 14 is also shown in FIG. 2, and includes an integrated circuit with memory for storing an identifier of an apparatus to which the tag 14 is attached, as well as a tag identifier. The passive RFID tag 14 also comprises one or more antennas. However, the passive RFID tags 14 contain no power source or transmitter. The passive RFID tag 14 may be embodied as an integrated circuit formed on a substrate, embodied as a printable RFID label, among other form factors. Operation of the passive RFID tag 14 may generally be in the low (e.g., 124-135 kHz), high (e.g., 13.56 MHz), or ultra-high frequency (UHF) range (e.g., 860-960 MHz, or higher). The low frequency RFID tags 14 generally operate with the identifier receiving circuit 52 according to inductive coupling, where the antenna 64 of the identifier receiving circuit 52 and the antenna of the RFID tag 14 form an electromagnetic field and the RFID tag 14 uses the electromagnetic field to draw power and change the electric load on the antenna. The identifier receiving circuit 52 senses the change in magnetic field and, through cooperation with the ADC 34, DSP 40, and microcontroller 38, converts this change to a digitized format. A passive RFID tag 14 operating in the UHF range uses propagation coupling, where the identifier receiving circuit 52 emits electromagnetic energy, but no (or no significant) electromagnetic field is created. Rather, the RFID tag 14 gathers energy from the signal provided by the identifier receiving circuit 52 and uses the energy to change the load on the antenna of the RFID tag 14 and reflect back an altered signal (also referred to as a back scattered signal). The signal may be modulated using amplitude, phase, or frequency modulation techniques. In propagation coupling, the antenna 64 is tuned to receive radio waves of a particular frequency. Processing of the backscattered signal at the identifier receiving circuit 52 undergoes a similar process as described above.

In one example operation, and assuming the control is performed by the microcontroller 38, the microcontroller 38 issues a command (e.g., periodically, or upon prompt by the subject) to read one or more of the passive (e.g., UHF) RFID tags 14. In one embodiment, the command issued by the microcontroller 38 may include the tag identifiers of the tags 14 for which a response signal is solicited, instructions for the processor of the tag 14, and/or information to be written to the tag 14 (where read/writeable). The command may be encoded by the DSP 40, and converted to analog (e.g., via DAC 36) for use by the transmitter circuit 60 and the signal generator circuit 68. Responsively, the signal generator circuit 68 generates a radio frequency signal, and based on the radio frequency signal generated by the signal generator circuit 68, the transmitter circuit 60 provides the RF signal to the directional coupler 62. In embodiments where multiple antennas 64 are implemented, an additional switch may be used to select the appropriate antenna 64 (e.g., as directed by the microcontroller 38). The transmitter circuit 60 initially provides a non-modulated, continuous wave signal to power up the passive RFID tag 14 or tags 14 that are within range, and then provides a modulated signal (e.g., amplitude modulated) with the command encoded therein. After a defined length of time of transmission of the modulated signal, the transmitter circuit 60 provides the continuous wave signal to enable backscattering of the signal by the one or more tags 14. The backscattered signal is received at the antenna 64, and then through the directional coupler 62, which directs the received signal to the receiver circuit 66. The receiver circuit 66 demodulates the received signal and passes the signal to the ADC 34, which digitizes the signal and passes the resulting digitized signal to the DSP 40 under the direction of the microcontroller 38. The DSP 40 decodes the digitized signal and passes the decoded information to the microcontroller 38. The microcontroller 38 access the data structure 50 (e.g., look-up-table or LUT) of the processing circuit 32 to compare the decoded information to a list of identifiers (activity or apparatus identifiers) to determine a match. Based on the match, the microcontroller 38 has identified the activity associated with the RFID tag 14, and responsively causes the ADC 34 to have an elevated sampling rate and deploys the appropriate algorithms 48 and sensors 28 (e.g., subset) for processing of physiological and/or behavioral parameters of the subject engaged in the detected activity.

In some embodiments, the identifier receiving circuit 52 may deploy additional functionality, such as anti-collision functionality (e.g., tag talk first or TTF) when multiple tags 14 are under interrogation. Although described above in the context of interrogating passive RFID tags 14, it should be appreciated that similar functionality may be performed for active or semi-active RFID tags 14. For instance, active RFID tags provide their own transmitter and a power source (e.g., battery, or according to reusable energy, such as solar conversion), enabling the tags 14 to broadcast the information (tag identifier and/or apparatus identifier) to the identifier receiving circuit 52. The broadcast of the information may be prompted by a wake-up signal (e.g., continuous wave signal) from the identifier receiving circuit 52, in which case the active RFID tag 14 is referred to as a transponder. Alternatively, the broadcast of the information may be implemented via a beacon provided from the active RFID tag 14, with the beacon containing the information stored in the memory of the active RFID tag 14 (e.g., tag identifier and/or apparatus identifier) and emitted by the active RFID tag 14 at predetermined intervals (e.g., every several seconds, though longer or shorter interval durations may be used).

In some embodiments, the identifier receiving circuit 52 may be embodied as an NFC circuit. The NFC transceiver and tags have overlapping functionality, and hence have similar hardware, the discussion of which is omitted here for brevity. For instance, NFC is a subset of RFID technology, and in particular, a branch of the high frequency RFID tag (e.g., both operating at 13.56 MHz). An NFC reader and tag use inductive coupling in a similar manner to an RFID reader and tag, and use either active or passive tags (though scans of a tag in NFC are achieved one at a time). The standards and protocols of the NFC format are based on the RFID standards outlined in ISO/IEC 14443. A device configured according to NFC is also capable of being both a tag and a reader, enabling peer-to-peer communications. NFC devices can read passive NFC tags, and some NFC devices can read passive RFID tags that are compliant with ISO 15693.

It should be appreciated that two or more of the functionality for the RFID, NFC, and Bluetooth receiver may be integrated as a single package (e.g., chip) with shared componentry in some embodiments of an identifier receiving circuit, and in some embodiments, a plurality of these transceiver circuits may be deployed.

When the identifier receiving circuit 52 is embodied as a Bluetooth transceiver, the circuit illustrated in FIG. 2 for the external communications circuit 54 may be used, although variations of the depicted circuit 54 may be implemented in some embodiments according to one having ordinary skill in the art in the context of the present disclosure. Description for the external communications circuit 54 follows, with the understanding that the same or similar architecture description applies for the identifier receiving circuit 52 implemented with Bluetooth technology for certain embodiments. As noted previously, the external communications circuit 54 is used to wirelessly interface with other electronic devices in the system 10 (FIG. 1). In one embodiment, the external communications circuit 54 may be configured as a Bluetooth transceiver, though in some embodiments, other and/or additional technologies may be used, such as Wi-Fi, Zigbee, NFC, among others. In the embodiment depicted in FIG. 2, the external communications circuit 54 comprises a transmitter circuit 70, a switch 72, an antenna 74, a receiver circuit 76, a mixing circuit 78, and a frequency hopping controller 80. The transmitter circuit 60 and the receiver circuit 66 comprise components suitable for providing respective transmission and reception of an RF signal, including a modulator/demodulator, filters, and amplifiers. In some embodiments, demodulation/modulation and/or filtering may be performed in part or in whole by the DSP 40. The switch 72 switches between receiving and transmitting mode. The mixing circuit 78 may be embodied as a frequency synthesizer and frequency mixers, as controlled by the processing circuit 32. The frequency hopping controller 80 controls the hopping frequency of a transmitted signal based on feedback from a modulator of the transmitter circuit 70. In some embodiments, functionality for the frequency hopping controller 80 may be implemented by the microcontroller 38 or DSP 40. Control for the external communications circuit 54 may be implemented by the microcontroller 38, the DSP 40, or a combination of both. In some embodiments, the external communications circuit 54 may have its own dedicated controller that is supervised and/or managed by the microcontroller 38.

In operation, a signal (e.g., at 2.4 GHz) may be received at the antenna 74 and directed by the switch 72 to the receiver circuit 76. The receiver circuit 76, in cooperation with the mixing circuit 78, converts the received signal into an intermediate frequency (IF) signal under frequency hopping control attributed by the frequency hopping controller 80 and then to baseband for further processing by the ADC 34. On the transmitting side, the baseband signal (e.g., from the DAC 36 of the processing circuit 32) is converted to an IF signal and then RF by the transmitter circuit 70 operating in cooperation with the mixing circuit 78, with the RF signal passed through the switch 72 and emitted from the antenna 74 under frequency hopping control provided by the frequency hopping controller 80. The modulator and demodulator of the transmitter and receiver circuits 70, 76, respectively, may be frequency shift keying (FSK) type modulation/demodulation, though not limited to this type of modulation/demodulation, which enables the conversion between IF and baseband. As noted previously, in some embodiments, demodulation/modulation and/or filtering may be performed in part or in whole by the DSP 40. The memory 42 stores firmware that is executed by the microcontroller 38 to control the Bluetooth transmission/reception.

Though several examples of communication technologies have been described above for the identifier receiving circuit 52, other and/or additional technologies utilizing a tag that provides coded identifying information using acoustic, optical, among other technologies, may be used in some embodiments. Some embodiments may use vibro-tactile technologies.

Though the external communications circuit 54 is depicted as an IF-type transceiver, in some embodiments, a direct conversion architecture may be implemented. As noted above, the external communications circuit 54 may be embodied according to other and/or additional transceiver technologies, such as NFC, Wi-Fi, or Zigbee. For instance, when Zigbee and/or Wi-Fi is deployed, which can operate in the 2.4 GHz RF range, a similar physical (PHY) layer architecture may be used (e.g., with the frequency hopping controller 80 replaced with a channel selector, and possibly a different modulation/demodulation scheme, such as minimum-shift keying (MSK), among others) and a media access control (MAC) layer, application layer, and/or network layer executed by the microcontroller 38 may be configured with suitable firmware/software (e.g., communications software 49) for enabling Zigbee or Wi-Fi transmission/reception control.

The processing circuit 32 is depicted in FIG. 2 as including the ADC 34 and DAC 36. For sensing functionality, the ADC 34 converts the conditioned signal from the signal conditioning circuit 30 and digitizes the signal for further processing by the microcontroller 38 and/or DSP 40. The sampling rate of the ADC 34 may be varied based on instructions from the microcontroller 38. For instance, the microcontroller may change (e.g., increase) the sampling rate upon detection of a signal (e.g., by the identifier receiving circuit 52) that is coded with information (e.g., an identifier) of an apparatus used by a subject, such as an identifier for a tennis racquet with an RFID tag 14 attached thereto. During periods of relative inactivity, the sampling rate may be lowered. The ADC 34 may also be used to convert analogs inputs that are received via the input interface 56 to a digital format for further processing by the microcontroller 38. The ADC 34 may also be used in baseband processing of signals received via the identifier receiving circuit 52 and/or the external communications circuit 54. The DAC 36 converts digital information to analog information. Its role for sensing functionality may be to control the emission of signals, such as optical signals or acoustic signal, from the sensors 28. The DAC 36 may further be used to cause the output of analog signals from the output interface 58. Also, the DAC 36 may be used to convert the digital information and/or instructions from the microcontroller 38 and/or DSP 40 to analog signal that are fed to the transmitter circuits 60 and 70. In some embodiments, additional conversion circuits may be used.

The microcontroller 38 and the DSP 40 provide the processing functionality for the wearable device 12. In some embodiments, functionality of both processors 38 and 40 may be combined into a single processor, or further distributed among additional processors. The DSP 40 provides for specialized digital signal processing, and enables an offloading of processing load from the microcontroller 38. The DSP 40 may be embodied in specialized integrated circuit(s) or as field programmable gate arrays (FPGAs). In one embodiment, the DSP 40 comprises a pipelined architecture, with comprises a central processing unit (CPU), plural circular buffers and separate program and data memories according to a Harvard architecture. The DSP 40 further comprises dual busses, enabling concurrent instruction and data fetches. The DSP 40 may also comprise an instruction cache and I/O controller, such as those found in Analog Devices SHARC® DSPs, though other manufacturers of DSPs may be used (e.g., Freescale multi-core MSC81xx family, Texas Instruments C6000 series, etc.). The DSP 40 is generally utilized for math manipulations using registers and math components that may include a multiplier, arithmetic logic unit (ALU, which performs addition, subtraction, absolute value, logical operations, conversion between fixed and floating point units, etc.), and a barrel shifter. The ability of the DSP 40 to implement fast multiply-accumulates (MACs) enables efficient execution of Fast Fourier Transforms (FFTs) and Finite Impulse Response (FIR) filtering. The DSP 40 generally serves an encoding and decoding function in the wearable device 12. For instance, encoding functionality may involve encoding commands or data corresponding to subject activity for triggering activation and operation of tags 14 and transfer of information to other electronic devices. Also, decoding functionality may involve decoding the information received from the tags 14 or from the sensors 28 (e.g., after processing by the ADC 34).

The microcontroller 38 comprises a hardware device for executing software/firmware (hereinafter, collectively, software 46 and 49), particularly that stored in memory 42. The microcontroller 38 can be any custom made or commercially available processor, a central processing unit (CPU), a semiconductor based microprocessor (in the form of a microchip or chip set), a macroprocessor, or generally any device for executing software instructions. Examples of suitable commercially available microprocessors include Intel's® Itanium® and Atom® microprocessors, to name a few non-limiting examples. The microcontroller 38 provides for management and control of the wearable device 12, including managing the detection of activity from the tags 14, determining physiological parameters based on the sensors 28, and for enabling communication with other electronic devices.

The memory 42 can include any one or combination of volatile memory elements (e.g., random access memory (RAM, such as DRAM, SRAM, SDRAM, etc.)) and nonvolatile memory elements (e.g., ROM, hard drive, tape, CDROM, etc.). Moreover, the memory 42 may incorporate electronic, magnetic, and/or other types of storage media.

The software in memory 42 may include one or more separate programs, each of which comprises an ordered listing of executable instructions for implementing logical functions. In the example of FIG. 2, the software in the memory 42 includes a suitable operating system (O/S) 44 and an application software 46 that includes a plurality of algorithms 48 (e.g., 48A, 48B, ...48N) for determining physiological and/or behavioral measures and/or other information (e.g., such as location) based on the selected subset of sensors 28. The raw data from the sensors 28 may be used by the algorithms 48 to determine various physiological and/or behavioral measures (e.g., heart rate, biomechanics, such as swinging of the arms), and may also be used to derive other parameters, such as energy expenditure, hear rate recovery, aerobic capacity (e.g., VO2 max, etc.), among other derived measures of physical performance. For instance, an algorithm for VO2 max may embodied as the equation 116.2-2.98*T-0.11*fh-0.14*A-0.39*BMI (for women, with a similar algorithm for men), where T is time, fh is final heart rate, A is age, and BMI is body mass index. As another example algorithm, exercise intensity may be a function of speed (e.g., stride (meters/step) number of steps per unit of time) multiplied by the weight of the subject. As another example of an algorithm corresponding to arm or hand movement, sensors (e.g., motion detectors) may detect each downswing, and generate signals that are processed (e.g., Fast Fourier transformed) and converted to determine, for instance, swings per unit time or power. Another example algorithm may be as simple as detecting heart rate by counting pulses detected by the sensors over a defined duration. Another example algorithm, such as for determining BMI, may be based on sensor data corresponding to bioelectrical impedance analysis and user data, such as weight. A basal metabolic rate algorithm may be embodied (e.g., for men) as 66+(13.7*weight) + (5*height) -(6.8*age), or the total daily energy expenditure is a function of the basal metabolic rate times an activity factor that varies depending on the level of activity or sedentary behavior of the subject. Note that these are just a few examples, and are not intended to be limiting. For instance, estimates of VO2 max may be according to an algorithm embodied as (D-504.9)/44.73, where D is the distance run in meters. These and other algorithms may be executed by the microcontroller 38 or DSP 40, and used as a basis for more specific activity-based determinations. The application software 46 may also include the communications software 49 to enable communications with other electronics devices. The operating system 44 essentially controls the execution of other computer programs, such as the application software 46 and communications software 49, and provides scheduling, input-output control, file and data management, memory management, and communication control and related services. The memory 42 also includes a data structure 50, which includes an association between the apparatus detected from the tag 14 in contact with the apparatus and the corresponding activity. The data structure 50 may be a database, linked list, LUT, among other types of data structures. The data structure 50 (or in some embodiments, another data structure in memory 42) may also include user data, such as weight, height, body mass index (BMI) that is used by the microcontroller 38 executing the executable code of the algorithm 48 to accurately interpret the measured physiological and/or behavioral data.

The software 46, 48, and 49 comprise a source program, executable program (object code), script, or any other entity comprising a set of instructions to be performed. When a source program, then the program needs to be translated via a compiler, assembler, interpreter, or the like, so as to operate properly in connection with the operating system 44. Furthermore, the software 46, 48, and 49 can be written as (a) an object oriented programming language, which has classes of data and methods, or (b) a procedure programming language, which has routines, subroutines, and/or functions, for example but not limited to, C, C+ +, Python, Java, among others. The software 46, 48, and 49 may be embodied in a computer program product, which may be a non-transitory computer readable medium or other medium.

The input interface 56 comprises an interface for entry of user input, such as a button or microphone or sensor (e.g., to detect user input). The input interface 56 may serve as a communications port for downloaded information to the wearable device 12 (such as via a wired connection). The output interfaces 58 comprises an interface for the presentation or transfer of data, such as a display or communications interface for the transfer (e.g., wired) transfer of information stored in the memory 42, or to enable one or more feedback devices, such as lighting devices (e.g., LEDs), audio devices (e.g., tone generator and speaker), and/or tactile feedback devices (e.g., vibratory motor). For instance, a vibratory motor of the wearable device 12 may be enabled concurrently with the change in sampling rate upon detection of the activity engaged in by the subject. In some embodiments, functionality of at least some of the functionality of the input and output interfaces 56 and 58 may be combined.

In operation, the microcontroller 38 executes software 46-49 stored within the memory 42, to communicate data to and from the memory 42, and to generally control operations of the wearable device 12 pursuant to the software. For instance, responsive to determining the identity of the activity in which the subject is engaged, the microcontroller 38 instructs (e.g., directly, or via the DSP 40) the ADC 34 to change the sampling rate and, in some embodiments, signals the output interface 58 to provide feedback to the subject about the detected activity (suggesting to the subject that physiological determinations are set to begin). The microcontroller 38 uses the information about the activity to be selective as to the sensors 28 for which input is desired. For instance, if the microcontroller 38 determines that the tag 14 is attached to a pillow, then the microcontroller 38 determines that the detected activity is rest or sleep, and may signal sensors 28 corresponding to heart rate, blood pressure, etc., but may omit sensors 28 such as a GPS sensor. In other words, the microcontroller 38 may utilize only a subset of the available sensors 28 to measure the physiological and/or biological parameters. In some embodiments, accuracy and/or refresh or other parameters of the sensors 28 may be adjusted depending on the detected activity. As another example, if the detected apparatus is a golf club (e.g., where a tag 14 is affixed to the club), the microcontroller 38 determines that body movements (e.g., behavioral parameters) and physiological parameters are to be associated with the golf swing, and data from a GPS sensor 28 and possibly climate sensors 28 may be selected for processing. The microcontroller 38 further uses the information about the activity to improve the interpretation of the data, such as through selective engagement of one of the plurality of algorithms 48 based on the determined activity. For instance, rhythm (or cadence) is a factor relevant for many different sports activities (e.g., cycling, running, swimming, rowing, skating, etc.), especially in combination with breathing rate. For the detected activity, the algorithm 48 is selected as appropriate for consideration of rhythm for that activity and breathing rate determination (e.g., the optimal breathing rate and rhythm may be different for each activity, and even for sub-types of the activity, such as sprinting versus long-distance running). As another example, derived parameters such as energy expenditure (e.g., basal metabolic rate x an activity factor based on the activity-specific data) may vary depending on, among other factors, the type of activity in which the subject is engaged (e.g., lifting 100 kilograms versus 20 kilograms, rowing a boat versus swing a golf club, etc.). By selecting an activity-specific algorithm for, say, energy expenditure computations, a more accurate determination of the energy expenditure may be determined. Other types of activity-specific information include those associated with the apparatus to which the tag 14 is affixed. For instance, for racquet sports, such as tennis, the algorithms 48 executed by the microcontroller 38 may determine the total amount of times the ball has been hit during a session, the amount of high-intensity hits or hits of a specific type, and/or total distance covered. Playing tennis comprises high intensity intervals (e.g., points lasting 5-30 seconds with a few hits per rally) alternated with low intensity intervals (preparation for the next point). Arm movement and the relationship between art movements and foot steps are different for the different intervals. That is, for instance, the behavioral parameters associated with arm movements during points (e.g., balancing the racquet, keeping arms stable, swinging the racquet) are different from movements in between the rallies (e.g., walking to a starting position for the next point). Such differentiation in movement is taken into account by the microcontroller 38 and selective algorithms 48 and sensors 28 for more accurate determinations of total distance covered during a match.

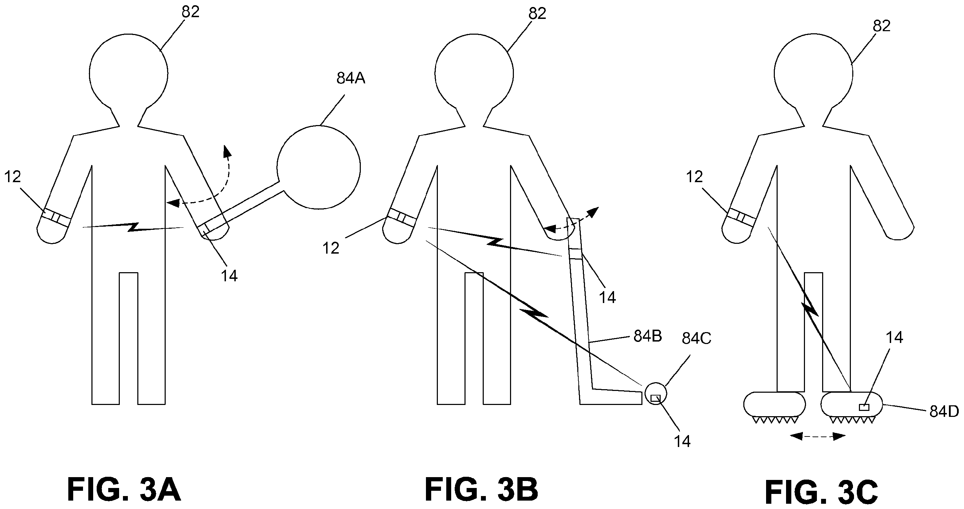

Having described the underlying hardware and software of the wearable device 12, attention is now directed to FIGS. 3A-3C, which illustrate how the wearable device 12 communicates (e.g., as represented in each of the FIGS. 3A-3C as well as other figures with a jagged "signal" line disposed between the tag 14 and wearable device 12) with the tag 14 to determine an activity engaged in by a subject. Reference to a subject (e.g., person) in the description that follows assumes the person is facing the reader of this document. Additionally, the examples illustrated in FIGS. 3A-3C are merely for illustrative purposes, and it should be understood that other variations for tag or wearable device placement or quantity of tags are contemplated. Referring to FIG. 3A, the subject 82 is wearing a wearable device 12 (e.g., a bracelet or band) around the wrist of his right arm and holding an apparatus 84A in his left hand. The apparatus 84A is depicted as a tennis racquet, though other apparatuses, such as a golf club, baseball bat, sword, among others may be used. The person is swinging the apparatus 84A back and forth, as represented symbolically by the dashed dual-headed arrow proximal to the left arm of the person 82. The apparatus 84A comprises a tag 14 attached to the handle of the apparatus 84A, though other locations for the tag 14 (or additional tags) along the apparatus 84A may be selected. The tag 14 may have an adhesive (e.g., passive RFID labels) that enables attached to the apparatus, though other mechanisms may be used, such as through embedding of the tag 14 into the structure of the apparatus or methods of securement (e.g., taped, stapled, etc.). In this and other implementations, the tag 14 and the wearable device 12 are disposed relative to each other up to a relatively short distance (e.g., 1-2 meters, and in this example, less than a meter). As long as the tag 14 is detected by the wearable device 12, the activity engaged in by the person (in this example, tennis) can be automatically attributed to tennis. As described previously, a short-range, wireless link between the wearable device 12 and the tag 14 attached to the apparatus 84A can be established using near-proximity type communications, such as RFID, NFC, and/or Bluetooth technology. The wearable device 12 measures the behavioral activity associated with swinging the apparatus back and forth, foot movement during, say, high and low intensity intervals, as well as a plurality of physiological parameters (e.g., heart rate, perspiration, respiration, blood pressure, skin temperature, muscle activity, etc.). Note that, in some embodiments, the microcontroller 38 (FIG. 2) of the wearable device 12 also assesses whether the wearable device 12 is worn on the dominant or non-dominant hand to facilitate accurate feature extraction (e.g., relevant for activities where the apparatus is in one hand, such as a racquet, golf club, walking stick, etc.).

The wearable device 12, through the sensing of activity, can also automatically initiate monitoring of the swinging activity and select-physiological parameters (e.g., using select-sensors 28 and/or algorithms 48) until activity completion, providing a more accurate assessment of the activity and associated physiological and/or behavioral impact. In other words, as an example, the detection of the tag 14 causes the microcontroller 38 of the wearable device 12 to change the sampling (e.g., cause the ADC 34 to increase in sampling rate, enabling more frequent or accurate data acquisition) and further selectively switch on certain sensory functions (e.g., a subset of the sensors 28) in the wearable device 12 that are needed to accurately measure the activity. A typical example of switched on sensors specific to the activity in this example may be a PPG sensor for heart rate and possibly relative blood pressure sensing. In some embodiments, the detection of the tag 14 (the determination of the activity) activates an appropriate algorithm 48 (FIG. 2) to measure parameters specific to the activity, such as to provide feedback about certain characteristics of the activity. For instance, the wrist-worn wearable device 12 may be used to distinguish and measure different movements associated with the type of activity, such as types of swings (e.g., backhand versus forehand) during the playing of tennis. Similar principles apply to other activities, such as distinguish and measure different types of strokes (breaststroke versus butterfly) during a swimming activity. Related to this feature is the capability of the wearable device 12 to, during analysis of the activity related parameters and other physiological and/or behavioral activity, use the data to distinguish between an initiation of activity versus activities surrounding the activity. For instance, the microcontroller 38 may begin recording the actual activity level, heart rate, and/or other physiological and/or behavioral parameter after these parameters have met or exceeded a predetermined threshold level that correlates to the detected activity (as opposed to walking with the racquet to the tennis court). In other words, the exceeding or meeting of the threshold level is indicative of actual activity unique to the type of activity. Such a feature not only improves accuracy but also reduces the consumption of power by the wearable device 12. Other examples of an apparatus in which the tag 14 may be attached and detected include a helmet (e.g., for motor biking, biking, skiing, boarding), a surf board, a mountain bike or racing bike, row handle, boat/kayak, fitness equipment (e.g., weights, machines, etc.), gymnasium apparatuses (e.g., rings, bars, etc.). Further, the type of apparatus may also include those not typically attributed to sports or fitness activity, such as a headboard or pillow (e.g., to detect sleep activity), objects related to rehabilitation or injuries, such as a prosthesis, wheel chairs, crutches, or recreational such as a walking stick, etc. In some embodiments, the apparatus to which the tag 14 is affixed may be other objects, such as a pill box, candy container, cigarette lighter, or other habit or persistently-accessed objects.

Referring to FIG. 3B, the person 82 is engaging in an activity of field hockey. The wearable device 12 is worn on his right wrist, and the apparatus 84B (a field hockey stick) is held by the left hand of the person 82. Attached to the apparatus 84B is a tag 14, which provides a signal that is wirelessly received by the wearable device 12 and used, like in FIG. 3A, to determine the type of activity the person 82 is engaged in. In some embodiments, the tag 14 is integrated into a part of an apparatus that has only intermittent and indirect contact with the person 82. In the depicted example, the other apparatus 84C is a ball with a tag 14 attached to it. In some implementations, the ball may be replaced with tennis ball, soccer ball, spear, discus, Frisbee, dart, etc. that have a tag(s) attached thereto. In such embodiments, the microcontroller 38 may generate statistics related to the sensing of the proximity of the apparatus (ball) 84C, such as amount of "touches" or duration of possession, etc.

FIG. 3C is another example configuration, where the person 82 is wearing the wearable device 12 around his right wrist, and is also wearing an apparatus 84D in the form of soccer shoes with a tag 14 attached thereto. The tag 14 may be used similarly to the tag 14 on the ball, such as to generate statistics of soccer ball possession, or in some embodiments, to detect the kicking force or speed. Although shown on the shoes of the person, a similar principle applies to an apparatus that includes, for instance, gloves (e.g., baseball gloves, hockey gloves, weightlifting gloves, etc.), skates, ballet or gymnastic slippers, among other apparatuses worn by the person with tags 14 attached thereto.

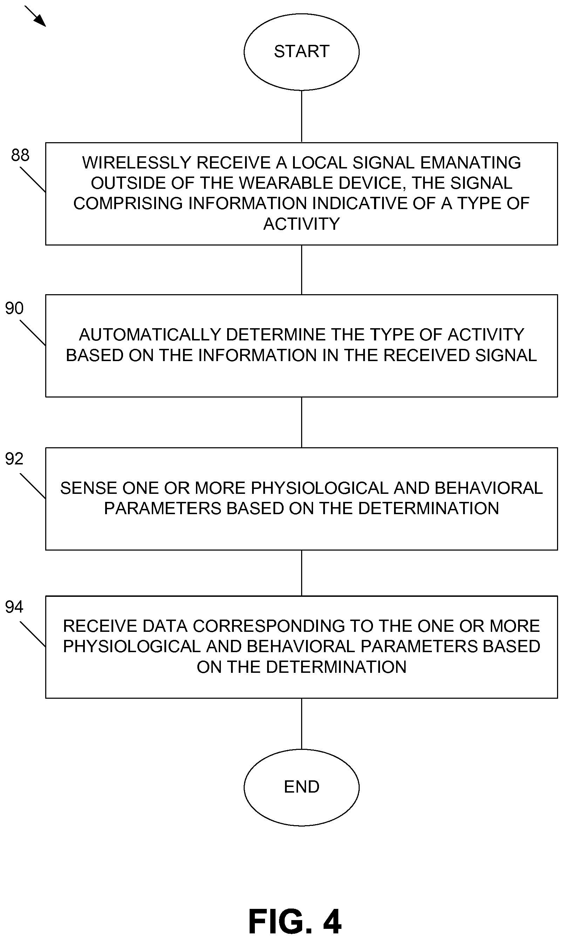

In view of the description above, it should be appreciated that one embodiment of a method for operating a wearable device, depicted in FIG. 4 and referred to as a method 86 and encompassed between the start and end designations, comprises wirelessly receiving a local signal emanating outside of the wearable device, the signal comprising information indicative of a type of activity (88); automatically determining the type of activity based on the information in the received signal (90); sensing one or more physiological and behavioral parameters based on the determination (92); and receiving data corresponding to the one or more physiological and behavioral parameters based on the determination (94).

Any process descriptions or blocks in the flow diagram of FIG. 4 should be understood as representing modules, segments, or portions of code which include one or more executable instructions for implementing specific logical functions or steps in the process, and alternate implementations are included within the scope of an embodiment of the present invention in which functions may be executed substantially concurrently, depending on the functionality involved, as would be understood by those reasonably skilled in the art of the present invention.

While the invention has been illustrated and described in detail in the drawings and foregoing description, such illustration and description are to be considered illustrative or exemplary and not restrictive; the invention is not limited to the disclosed embodiments. For example, it is possible to operate the invention in an embodiment wherein the identification of the activity may be achieved using proximity sensing based on conductivity of the skin or body as described in U.S. Patent 7,664,476 or U.S. Publication 20090124201. For instance, the tag 14 is in contact with the body (e.g., at the feet) and sends a modulated electric signal across the body, which is received by the wearable device 12 worn on the wrist. Further, though the manner of how the wearable device 12 is typically described above as worn on the wrist (e.g., as a bracelet or band), the wearable device 12 may be worn on other portions of the body directly or indirectly (e.g., through attachment to apparel worn by the person). As another example of an alternative embodiment, the wearable device 12 may make a further distinction between the type of species of apparatus among a generic type of apparatus. For instance, with golf, the swing dynamics may differ between, say, a nine-iron and a putter, and hence individual and distinctly identified tags 14 may be used for different species of apparatuses within a given genus. As another variation, although an embodiment of the wearable device 12 has been described as having memory 42 with a data structure 50 that associates the identifier information received from the tag 14 with the activity, in some embodiments, the wearable device 12 may access the memory (e.g., in the form of a SQL query or like event) of another electronic device (e.g., the phone 16, laptop 18, etc., functioning as a server in a client-server relationship to the wearable device 12) to determine the identity based on the coded identifier information (and hence not use local memory for such a data structure). The identification of an apparatus through wireless signals from a tag 14 enables additional applications beyond the use of the physiological and/or biological sensing. For instance, a tag 14 may be affixed to a cigarette lighter that, when identified by the microcontroller 38, enables the microcontroller 38 to prompt a questionnaire to help a subject understand situations and/or contexts in which the subject tends to smoke more. In some embodiments, a sensor 28 (e.g., GNSS) may be used to facilitate the context understanding. As another example, a tag 14 may be disposed in a kitchen area (e.g., affixed to a kitchen appliance), such that when in proximity to the wearable device 12 worn by the subject, triggers a questionnaire to determine the situation/context and/or requires the subject to take a weight measurement before eating. Other variations to the disclosed embodiments can be understood and effected by those skilled in the art in practicing the claimed invention, from a study of the drawings, the disclosure, and the appended claims. Note that various combinations of the disclosed embodiments may be used, and hence reference to an embodiment or one embodiment is not meant to exclude features from that embodiment from use with features from other embodiments. In the claims, the word "comprising" does not exclude other elements or steps, and the indefinite article "a" or "an" does not exclude a plurality. A single processor or other unit may fulfill the functions of several items recited in the claims. The mere fact that certain measures are recited in mutually different dependent claims does not indicate that a combination of these measures cannot be used to advantage. A computer program may be stored/distributed on a suitable medium, such as an optical medium or solid-state medium supplied together with or as part of other hardware, but may also be distributed in other forms. Any reference signs in the claims should be not construed as limiting the scope.

## Claims

1. A wearable device (12), comprising:
a wireless receiver circuit (66) for wirelessly receiving a local signal emanating outside of the wearable device (12), the signal comprising information indicative of a type of activity selectable from among a plurality of types of activities;
a plurality of sensors (28) for sensing one or more physiological and behavioral parameters;
**characterized in that** the local signal emanating outside of the wearable device (12) comprises an apparatus identifier, and the wearable device (12) associates the identifier of the apparatus with a specific activity;
a processing circuit (32) for automatically determining a type of activity based on the apparatus identifier and for receiving data corresponding to the one or more physiological and behavioral parameters from one or more of the plurality of sensors based on the determination.

2. The wearable device of claim 1, wherein the determined type of activity is related to a person (82) wearing the wearable device (12), and the one or more physiological and behavioral parameters are associated with the person's body function or movement of all or a part of the person's body.

3. The wearable device of claim 1, wherein the type of activity comprises type of sports activity, type of exercise activity, type of recreational activity, type of household activity, type of social activity, or type of sedentary activity.

4. The wearable device of claim 1, wherein the wireless receiver circuit (66) is for wirelessly receiving the signal coded with identifier information according to radio frequency identification technology, near field communication technology, or Bluetooth technology.

5. The wearable device of claim 1, wherein receiving data corresponding to the one or more physiological and behavioral parameters from one or more of the plurality of sensors based on the determination of the type of activity further comprises activating a subset of the plurality of sensors to measure the one or more physiological and behavioral parameters based on the determination of the type of activity.

6. The wearable device of claim 1, further comprising memory (42) for storing a data structure (50) that associates the information with an apparatus (84) or the type of activity associated with the apparatus.

7. The wearable device of claim 6, the processing circuit further being arranged for executing one of a plurality of selectable algorithms (48) stored in the memory (42) based on determining the type of activity.

8. The wearable device of claim 7, the processing circuit further being arranged for distinguishing and measuring different movements associated with the determined type of activity.

9. The wearable device of claim 1, the wireless receiver circuit (66) further being arranged for receiving the local signal from a tag (14) attached to an apparatus (84) that is in direct or indirect contact with a person (82).

10. The wearable device according to claim 9, wherein the tag is a passive RFID tag.

11. The wearable device according to claim 9, wherein the type of activity is determined based on the tag.

12. The wearable device of claim 1, the processing circuit (32) further being arranged for causing the provision of feedback responsive to determining the type of activity.

13. The wearable device of claim 1, the processing circuit (32) further being arranged for recording the one or more physiological and behavioral parameters based on the physiological and behavioral parameters meeting or exceeding a predetermined threshold level indicative of actual activity unique to the type of activity.

14. A method (86) of operating the wearable device (12) according to claim 1, comprising:
wirelessly receiving a local signal emanating outside of the wearable device, the signal comprising an apparatus identifier, the wearable device (12) associating the identifier of the apparatus with a specific activity;
automatically determining the type of activity based on the apparatus identifier (90);
sensing one or more physiological and behavioral parameters based on the determination (92); and
receiving data corresponding to the one or more physiological and behavioral parameters based on the determination (94).

15. A computer program product comprising computer program code means to cause the wearable device as claimed in claim 1, to receive the signal comprising the apparatus identifier, and to perform the steps of the method of claim14.

## Patentansprüche

1. Ein tragbares Gerät (12), das aus Folgendem besteht:
einer drahtlosen Empfängerschaltung (66) zum drahtlosen Empfangen eines lokalen, von außerhalb des des tragbaren Geräts (12) ausgehenden Signals, wobei das Signal aus Daten besteht, die auf eine Art von Aktivität hinweisen, die unter ein Vielzahl von Aktivitcätsarten ausgewählt werden kann;
mehreren Sensoren (28) zum Erkennen mindestens eines physiologischen und Verhaltensparameters;
wobei das außerhalb des tragbaren Geräts (12) ausgehende lokale Signal eine Geräte-ID umfasst, die vom tragbaren Gerät (12) einem Gerät mit einer bestimmten Aktivität zuordnet wird;
einer Verarbeitungsschaltung (32) zum automatischen Erkennen einer Aktivität beruhend auf der Geräte-ID sowie zum Empfangen von Daten, die im Rahmen der Erkennung mindestens einem physiologischen und Verhaltensparameter von einem der Sensoren entspricht.

2. Das tragbare Gerät gemäß Anspruch 1, wobei sich die erkannte Art der Aktivität auf die Person (82) bezieht, die das tragbare Gerät (12) mit sich führt. Hierbei wird mindestens ein physiologischer und Verhaltensparameter der Körperfunktion der Person oder der von Körperteilen oder dem gesamten Körper der Person ausgehenden Bewegungen zugeordnet.

3. Das tragbare Gerät gemäß Anspruch 1, wobei die erkannte Art der Aktivität sportliche, Trainings-, Freizeit-, Haushalts-, soziale sowie sitzende Aktivitäten umfasst.

4. Das tragbare Gerät gemäß Anspruch 1, wobei die drahtlose Empfängerschaltung (66) zum drahtlosen Empfangen des mit den ID-Daten codierten Signals gedacht ist. Dies erfolgt mithilfe von Funkfrequenzerkennungs-, Nahbereichskommunikations- oder Bluetooth-Technologien.

5. Das tragbare Gerät gemäß Anspruch 1, wobei das Empfangen von Daten über mindestens einen physiologischen und Verhaltensparameter von mindestens einem der Sensoren beruhend auf dem Erkennen der Art der Aktivität zudem das Aktivieren mehrerer zusätzlicher Sensoren umfasst, um beruhend auf der erkannten Art der Aktivität mindestens einen physiologischen und Verhaltensparameter zu messen.

6. Das tragbare Gerät gemäß Anspruch 1, wobei dieses zudem einen Speicher (42) zum Speichern einer Datenstruktur (50) umfasst, im Rahmen derer die Daten einem Gerät (84) oder der diesem Gerät entsprechenden Art der Aktivität zugeordnet werden.

7. Das tragbare Gerät gemäß Anspruch 6, wobei die Verarbeitungsschaltung zudem so eingerichtet ist, dass beruhend auf der erkannten Art der Aktivität einer von mehreren, im Speicher (42) gespeicherten optionalen Algorithmen (48) ausgeführt wird.

8. Das tragbare Gerät gemäß Anspruch 7, wobei die Verarbeitungsschaltung zudem so eingerichtet ist, dass beruhend auf der erkannten Art der Aktivität verschiedene Bewegungen gemessen und unterschieden werden.

9. Das tragbare Gerät gemäß Anspruch 1, wobei die drahtlose Empfängerschaltung (66) zudem so eingerichtet ist, dass das lokale Signal eines am Gerät (84) angebrachten Tags (14) empfangen wird,
der sich in direktem oder indirektem Kontakt mit einer Person (82) befindet.

10. Das tragbare Gerät gemäß Anspruch 9, wobei es sich beim Tag um einen passiven RFID-Tag handelt.

11. Das tragbare Gerät gemäß Anspruch 9, wobei die Art der Aktivität anhand des Tags ermittelt wird.

12. Das tragbare Gerät gemäß Anspruch 1, wobei die Verarbeitungsschaltung (32) zudem so eingerichtet ist, dass beim Erkennen der Art der Aktivität ein Feedback gesendet wird.

13. Das tragbare Gerät gemäß Anspruch 1, wobei die Verarbeitungsschaltung (32) zudem so eingerichtet ist, dass mindestens ein physiologischer oder Verhaltensparameter abhängig davon aufgezeichnet wird, ob der physiologische oder Verhaltensparameter einen vordefinierten Schwellenwert erreicht oder überschreitet, der die vorliegende Art der Aktivität eindeutig angibt.

14. Ein Verfahren (86) für
den Betrieb des tragbaren Geräts (12) gemäß Anspruch 1,
das Folgendes umfasst:
drahtloses Empfangen eines lokalen Signals, das außerhalb des tragbaren Geräts ausgeht, wobei das Signal eine Geräte-ID umfasst, die vom tragbaren Gerät (12) einem Gerät mit einer bestimmten Aktivität zugeordnet wird;
automatisches Erkennen der Art der Aktivität beruhend auf der Geräte-ID (90);
Wahrnehmen von mindestens einem physiologischen und Verhaltensparameter beruhend auf der Erkennung (92); und
Empfangen von Daten über mindestens einen physiologischen und Verhaltensparameter beruhend auf der Erkennung (94).

15. Ein Computerprogramm, das mithilfe von Computerprogrammcode das tragbare Gerät gemäß Anspruch 1 dazu veranlasst,
das Signal mit der Geräte-ID zu empfangen und die Schritte des
Verfahrens gemäß Anspruch 14 durchzuführen.

## Revendications

1. Dispositif portable (12) comprenant :
un circuit récepteur sans fil (66) pour recevoir par voie hertzienne un signal local provenant de l'extérieur du dispositif portable (12), le signal comprenant des informations représentatives d'un type d'activité choisie parmi une pluralité de types d'activités ;
une pluralité de capteurs (28) pour capter un ou plusieurs paramètres physiologiques et comportementaux ;
**caractérisé en ce que** le signal local provenant de l'extérieur du dispositif portable (12) comprend un identifiant d'appareil, et le dispositif portable (12) associe l'identifiant d'appareil à une activité spécifique ;
un circuit de traitement (32) pour déterminer automatiquement un type d'activité sur la base de l'identifiant d'appareil et pour recevoir des données correspondant à un ou plusieurs de la pluralité de capteurs sur la base de la détermination.

2. Dispositif portable selon la revendication 1, dans lequel le type d'activité déterminé est associé à une personne (82) portant le dispositif portable (12), et un ou plusieurs paramètres physiologiques ou comportementaux sont associés à la fonction corporelle de la personne ou à un mouvement d'une partie ou de tout le corps de la personne.

3. Dispositif portable selon la revendication 1, dans lequel le type d'activité comprend le type d'activité sportive, le type d'activité physique, le type d'activité récréative, le type d'activité domestique, le type d'activité sociale, ou le type d'activité sédentaire.

4. Dispositif portable selon la revendication 1, dans lequel le circuit récepteur sans fil (66) est destiné à recevoir par voie hertzienne le signal codé avec les informations d'identifiant selon la technologie d'identification de fréquence radio, la technologie de communication NFC, ou la technologie Bluetooth.

5. Dispositif portable selon la revendication 1, dans lequel les données de réception correspondant à un ou plusieurs paramètres physiologiques et comportementaux à partir d'un ou plusieurs de la pluralité de capteurs sur la base de la détermination du type d'activité comprend en outre l'activation d'un sous-ensemble de la pluralité de capteurs pour mesurer un ou plusieurs paramètres physiologiques et comportementaux sur la base de la détermination du type d'activité.

6. Dispositif portable selon la revendication 1, comprenant en outre une mémoire (42) pour stocker une structure de données (50) qui associe les informations avec un appareil (84) ou le type d'activité associé à l'appareil.

7. Dispositif portable selon la revendication 6, le circuit de traitement étant en outre agencé pour exécuter un parmi une pluralité d'algorithmes sélectionnables (48) stockés dans la mémoire (42) sur la base de la détermination du type d'activité.

8. Dispositif portable selon la revendication 7, le circuit de traitement étant en outre agencé pour distinguer et mesurer différents mouvements associés au type d'activité déterminé.

9. Dispositif portable selon la revendication 1, le circuit récepteur sans fil (66) étant en outre agencé pour recevoir le signal local à partir d'une étiquette (14) fixée à un appareil (84) en contact direct ou indirect avec une personne (82).

10. Dispositif portable selon la revendication 9, dans lequel l'étiquette est une étiquette RFID passive.

11. Dispositif portable selon la revendication 9, dans lequel le type d'activité est déterminé en se basant sur l'étiquette.

12. Dispositif portable selon la revendication 1, le circuit de traitement (32) étant en outre agencé pour pouvoir fournir des informations en vue de déterminer le type d'activité.

13. Dispositif portable selon la revendication 1, le circuit de traitement (32) étant en outre agencé pour enregistrer un ou plusieurs paramètres physiologiques et comportementaux sur la base des paramètres physiologiques et comportementaux atteignant ou dépassant un niveau seuil prédéterminé de l'activité réelle unique au type d'activité.

14. Procédé (86) de fonctionnement
du dispositif portable (12) selon la revendication 1, comprenant :
la réception par voie hertzienne d'un signal local provenant de l'extérieur du dispositif portable, le signal comprenant un identifiant d'appareil, le dispositif portable (12) associant l'identifiant d'appareil à une activité spécifique ;
la détermination automatique du type d'activité sur la base de l'identifiant d'appareil (90) ;
la détection d'un ou de plusieurs paramètres physiologiques et comportementaux sur la base de la détermination (92) ; et
la réception des données correspondantes à un ou plusieurs des paramètres physiologiques et comportementaux sur la base de la détermination (94).

15. Produit de programme informatique comprenant un moyen de code de programme informatique permettant au dispositif portable selon la revendication 1, de recevoir
le signal comprenant l'identifiant d'appareil, et d'exécuter
les étapes du procédé de la revendication 14.
